(19) Europäisches Patentamt
European Patent Office
Office européen des brevets

(11) Publication number: **0 068 877**
**B1**

(12) # EUROPEAN PATENT SPECIFICATION

(45) Date of publication of patent specification: **29.07.87**

(51) Int. Cl.$^4$: **C 07 D 345/00,** G 03 G 5/09

(21) Application number: **82303383.2**

(22) Date of filing: **28.06.82**

(54) **Telluropyrylium compounds and electron donating photoconductive compositions containing same.**

(30) Priority: **01.07.81 US 279365**

(43) Date of publication of application:
**05.01.83 Bulletin 83/01**

(45) Publication of the grant of the patent:
**29.07.87 Bulletin 87/31**

(84) Designated Contracting States:
**BE DE FR GB**

(56) References cited:
**EP-A-0 068 874**

**Chemical Abstracts vol. 96, no. 13, 29 March 1982, Columbus, Ohio, USA M.R. DETTY et al. "Delta 4',4'-4-Telluropyranyl-4H-telluropyrans. 1. Tellurosulfides and tellurium-sulfur exchange", page 691, column 1, abstract no. 104060t**

**Chemical Abstracts vol. 96, no. 21, 24 May 1982, Columbus, Ohio, USA M.R. DETTY et al. "Preparation of 2,6-diphenyl-4H-chalcogenapyran-4-ones", page 695, column 1, abstract no. 181104n**

(73) Proprietor: **EASTMAN KODAK COMPANY**
**343 State Street**
**Rochester New York 14650 (US)**

(72) Inventor: **Detty, Michael Ray**
**132 Burwell Road**
**Rochester New York 14617 (US)**
Inventor: **Murray, Bruce John**
**196 Holleybrook Road**
**Brockport New York 14420 (US)**
Inventor: **Perlstein, Jerome Howard**
**65 Runnymede Road**
**Rochester New York 14618 (US)**

(74) Representative: **Baron, Paul Alexander Clifford et al**
**Kodak Limited Patent Department**
**Headstone Drive**
**Harrow Middlesex HA1 4TY (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 068 877 B1

**Description**

This invention relates to telluropyrylium compounds and to their utility as electron accepting dye sensitizers in electron donating photoconductive compositions.

U.S. Patent 3,971,742 discloses the use of organic, polymeric, diselenide compounds into which is fused metallic tellurium in order to enhance flexibility, abrasion resistance and adhesive properties of imaging layers used in photoconductive compositions. There is no indication in this patent that the modified diselenide compounds have any utility as electron acceptors in electron donating photoconductive compositions.

Compounds comprising telluropyrylium nuclei, or starting materials with which to synthesize such telluropyrylium compounds, are not known in the prior art.

The present invention provides compounds which comprise a telluropyrylium nucleus, including a benzotelluropyrylium nucleus. These compounds are useful as electron acceptors in increasing the sensitivity of organic photoconductive compositions containing electron donating photoconductors.

Compounds of this invention comprise a telluropyrylium nucleus having the structural formula:

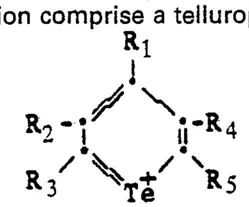 $(X^-)_m$; I.

wherein

$R_1$, $R_3$ and $R_5$ each independently represent a hydrogen atom or alkyl, alkylamino, dialkylamino, alkoxy, alkylaryl, aryl or aryloxy group where each alkyl or alkoxy is a substituted or unsubstituted, branched or straight-chain hydrocarbon having from 1 to 16 carbon atoms; and each aryl or aryloxy is a substituted or unsubstituted phenyl, naphthyl or anthryl group; or a heterocyclic group having from 5 to 20 carbon atoms where the hetero atoms are oxygen, phosphorus, nitrogen, sulfur, selenium or tellurium; or a

$$-(CH{=}CH)_n-CH{=}\underline{A}_1 \text{ or } -(CH{=}CH)_n-CH{=}\underline{A}_2 \text{ group,}$$

or a zwitterionic diketonate of the structural formula:

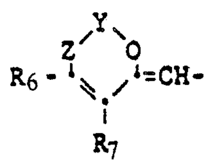 ;

$R_2$ and $R_4$ each independently represent a hydrogen atom or a substituted or unsubstituted, branched or straight-chain alkyl group having from 1 to 16 carbon atoms; or a substituted or an unsubstituted phenyl, naphthyl, or anthryl group; or

$R_2$ and $R_3$, or $R_4$ and $R_5$, taken together with the carbon atoms to which they are attached, form a fused mono- or polynuclear carbocyclic ring system having from 5 to 20 carbon atoms;

$R_6$ and $R_7$ each independently represent a hydrogen atom or a substituted or unsubstituted, branched or straight-chain alkyl group having from 1 to 16 carbon atoms; or a substituted or unsubstituted phenyl, naphthyl or anthryl group; or a substituted or unsubstituted heterocyclic group where the hetero atom is oxygen, phosphorus, nitrogen, sulfur, selenium or tellurium; or a mono- or polycyclic heterocyclylidene methyl group wherein the hetero atom is oxygen, phosphorus, nitrogen, sulfur, selenium or tellurium; or $R_6$ and $R_7$, taken together with the carbon atoms to which they are attached, form a fused mono- or polycyclic, carbocyclic or heterocyclic group having from 5 to 20 carbons wherein the hetero atoms is oxygen, phosphorus, nitrogen, sulfur, selenium or tellurium;

$\underline{A}_1$ represents a mono- or polycyclic heterocyclic group conjugated through the methine or the polymethine linking group, to the telluropyrylium ring wherein the hetero atoms are oxygen, phosphorus, nitrogen, sulfur, selenium or tellurium and the cyclic ring system has from 5 to 20 carbon atoms;

$\underline{A}_2$ represents a hydrogen atom, amino, alkyl, alkylamino, dialkylamino, dialkylaminoaryl or alkoxy group where each alkyl or alkoxy is a substituted or unsubstituted, branched or straight-chain hydrocarbon having from 1 to 16 carbon atoms; or substituted or unsubstituted aryl, arylamino or diarylamino group where each aryl is a phenyl, naphthyl or anthryl group; or a mono or polycyclic heterocyclic group having from 5 to 20 carbon atoms where the hetero atom can be oxygen, phosphorus, nitrogen, sulfur, selenium or tellurium, said heterocyclic group being conjugated, directly or indirectly through the polymethine linking group, to the telluropyrylium ring;

n is a number from 0 to 5;

m is 1, except when $R_1$, $R_3$ or $R_5$ is a zwitterionic group, then m is 0;

X is an anion;
Y is $BF_2$, or $PF_4$; and
Z is an oxygen or sulfur atom.

Other preferred dyes of this invention comprise a benzotelluropyrylium nucleus having the structural formula:

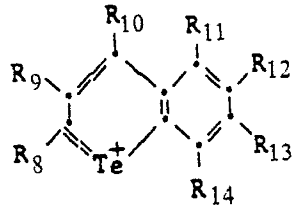

$(X^-)_{m'}$; III.

wherein

$R_8$ and $R_{10}$ each independently represent a hydrogen atom or an alkyl, alkylamino, arylamino, alkoxy, aryloxy, dialkylamino, diarylamino, $-(CH=CH)_{n'}-CH=A_{1'}$, or $-(CH=CH)_{n'}-A_2$ group;

$R_9$ represents a hydrogen atom or a substituted or unsubstituted, branched or straight-chain alkyl group having from 1 to 16 carbon atoms; or a substituted or an unsubstituted phenyl, naphthyl or anthryl group;

$R_{11}$, $R_{12}$, $R_{13}$ and $R_{14}$ each independently represent a hydrogen or a halogen atom or an alkyl, alkoxy, aryl, alkylamino or arylamino group; or

$R_{11}$ and $R_{12}$, $R_{12}$ and $R_{13}$ or $R_{13}$ and $R_{14}$, taken together with the carbon atoms to which they are attached, form a fused carbocyclic ring system having from 5 to 16 carbon atoms;

$A_1$ and $A_2$ are as defined above;

n' is 0, 1 or 2;

m' is 1

Additional preferred dyes of this invention comprise a telluropyrylium nucleus conforming to structural formula I wherein:

$R_1$, $R_3$ and $R_5$ each independently represent a hydrogen atom or a methyl, ethyl, phenyl, p-N,N-dimethylaminophenyl, p-anisyl, phenoxy, ethoxy, methoxy, $-(CH=CH)_{n'}-CH=A_1$ or $-(CH=CH)_{n'}-A_2$ group; or a zwitterionic diketonate having the structural formula:

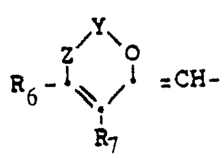

;

$R_6$ and $R_7$ each independently represent a methyl, phenyl, methoxyphenyl, p-N,N-dimethylaminophenyl, aminophenyl, pyridyl, oxazolyl, thiazolyl, selenazolyl, pyranyl, thiapyranyl, selenapyranyl, telluropyranyl, or oxaindolazinyl group or $R_6$ and $R_7$, taken together with the carbon atoms to which they are attached, are as defined above;

$A_1$ represents an oxazolylidene, thiazolylidene, selenazolylidene, imidazolylidene, pyranylidene, thiapyranylidene, selenapyranylidene, telluropyranylidene, oxaindolazinylidene benzoxazolyidene, benzothiazolylidene, benzopyranylidene, benzothiapyranylidene, benzoselenapyranylidene, or benzo-telluropyranylidene nucleus;

$A_2$ represents a hydrogen atom or a methyl, methoxy, ethoxy, phenyl, dimethylaminophenyl or dimethylamino group, or an oxazolyl, 9-julolidyl, pyridyl, thiazolyl, selenazolyl, imidazolyl, pyryliumyl, thiapyryliumyl, selenapyrylium, telluropyrylium, pyridinyl, furanyl, thiophenyl, selenophenyl, tellurophenyl, oxaindolazinyl, benzoxazolyl, benzothiazolyl, benzopyryliumyl, benzothiapyryliumyl, benzoselenapyryliumyl, benzotelluropyryliumyl or naphthyl nucleus; and

X represents $BF_4$, $ClO_4$, $SO_3$, $CF_3$, $CF_3SO_3$, $FSO_3$, $PF_6$, $CH_3SO_2$, chlorine, bromine or iodine.

Other preferred dyes of this invention comprise a benzotelluropyrylium nucleus conforming to structural Formula III wherein:

$R_8$ and $R_{10}$ each independently represent a hydrogen atom or a methyl, ethyl, methoxy, hydroxy, ethoxy, phenyl, phenoxy, p-anisyl, 2,5-dimethoxyphenyl, p-N,N-dimethylaminophenyl, diarylamino,

$-(CH=CH)_{n'}-CH=A_1$ or $-(CH=CH)_{n'}-A_2$ group;

$A_1$, $A_2$, $R_6$ and $R_7$ are as defined above for the preferred dyes according to Formula I; or $R_6$ and $R_7$, taken together with the carbon atom to which they are attached, form a fused naphthalene ring.

Heterocyclic and heterocyclidene groups can contain hetero atoms such as oxygen, phosphorus, nitrogen, sulfur, selenium or tellurium. Examples of the latter groups include those used to form cyanine dyes, such as pyridyl, furanyl, thiopyranyl, selenopyranyl, telluropyranyl, oxazolyl, thiazolyl, selenazolyl, tellurazolyl, benzoxazolyl, benzthiazolyl, benzselenazolyl and benztellurazolyl.

"Alkyl" as used herein refers to a branched- or straight-chain hydrocarbon having up to 16 carbon atoms, such as methyl, ethyl, propyl, isopropyl, butyl, isobutyl, nonyl or dodecyl. "Aryl" as used herein refers to phenyl, naphthyl and anthryl. Heterocyclic, heterocyclidene, alkyl and aryl are optionally further substituted with for example, allyl, aryl, halogen, nitro, cyano, carboxy, hydroxy, alkoxy, aryloxy, aralkyl, acyl, amide, sulfonamide, dialkylamino or amino groups.

The dyes of this invention are prepared with telluropyrone and benzotelluropyrone intermediates. The telluropyrone intermediates are prepared by admixing an alkyl alcohol solution containing a telluride dianion and an alkoxide anion with a solution containing a pentadiynone having the structural formula:

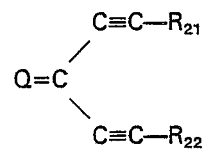

and the resulting intermediate has a mononuclear telluropyrone nucleus having the structural formula:

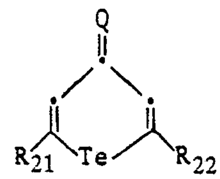

wherein $R_{21}$ and $R_{22}$ each independently can represent a halogen atom or an aryl, monocyclic or polycyclic heteroaromatic, alkyl, alkoxy, amino, trialkylsiloyl, triarylsilyl, alkylamino or dialkylamino group; and

Q represents an oxygen, sulfur or selenium atom.

Aryl, which is substituted or unsubstituted, includes phenyl, naphthyl, anthryl, methoxyphenyl, alkoxyphenyl, dialkylaminophenyl, alkylphenyl, nitrophenyl and halophenyl. Alkyl and "alkoxy" refer to branched- or straight-chain hydrocarbon groups having up to 16 carbon atoms, such as methyl, ethyl, propyl, isopropyl, butyl, isobutyl, nonyl and dodecyl. Mono- and polycyclic heteroaromatic groups refer to aromatic groups having hetero atoms such as oxygen, phosphorous, sulfur, nitrogen, selenium and tellurium. Examples of the latter groups include those used to form cyanine dyes, such as pyridyl, furaryl, thiophenyl, selenophenyl, tellurophenyl, oxazolyl, thiazolyl, selenazolyl, tellurazolyl, benzoxazolyl, benzthiazolyl, benzselenazolyl, and benztellurazolyl.

Substituents on alkyl, aryl and heteroaromatic groups include alkyl, aryl, halogen, nitro, cyano, carboxy, hydroxy, alkoxy, amido, aryl, amino, alkylamino, dialkylamino, trialkylsilyl, alkylarylsilyl, triarylsilyl, alkylthio, arylthio, and aryloxy.

The benzotelluropyrone intermediates are prepared by cyclizing a 3-aryltelluroacrylic acid or a 3-aryltelluroacryloyl halide, wherein

a) the aryltelluroacrylic acid or 3-aryltelluroacryloyl halide has the structural formula:

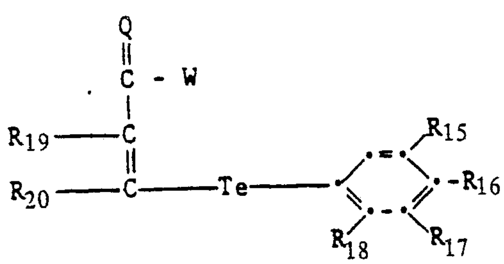

and

b) the resulting benzotelluropyrone has the structure:

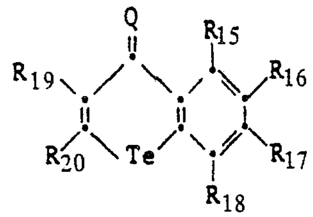

wherein

$R_{19}$ and $R_{20}$ each independently can represent a hydrogen or a halogen atom or an alkyl, alkoxy, or aryl group, or $R_{19}$ and $R_{20}$, together with the carbon atoms to which they are attached, form a fused carbocyclic or heterocyclic ring structure having 5 to 20 carbon atoms;

$R_{15}$ and $R_{17}$ each independently can represent an electron donating substituent such as a hydrogen or a halogen atom, or a hydroxy, alkoxy, aryloxy, amino, dialkylamino, alkylazo, arylazo, alkylthio or arylthio group;

$R_{16}$ and $R_{18}$ each independently can represent a hydrogen or a halogen atom or an alkyl group, or $R_{16}$ and $R_{17}$, or $R_{17}$ and $R_{18}$, together with the carbon atoms to which they are attached, can form a fused mono- or polycyclic, carbocyclic or heterocyclic ring structure having 5 to 20 carbon atoms;

Q is oxygen, sulfur or selenium atom and

W is OH, bromine, chlorine or iodine atom.

The starting materials are cyclized by contact with a Friedel-Crafts catalyst in a halogenated solvent such as methylene chloride, preferably in an inert atmosphere. The temperature of the solution is maintained at or below 0°C. From 0.1 to 1.1 equivalents of the selected Friedel-Crafts catalyst are added to the solution. The temperature of the solution is raised to 20°C to 40°C to allow the reaction to proceed to formation of the novel benzotelluropyrone compounds, including benzotelluropyrone thione compounds. After the reaction is completed the mixture is cooled to room temperature.

The starting materials are also cyclized by contact with a solution of phosphorous pentoxide in methane sulfonic acid. The contact is carried out by adding a solution consisting of from 5 to 10 percent by weight of phosphorus pentoxide in methane sulfonic acid to form 0.1 to 0.5 molar equivalents of the starting materials.

The telluropyrone and benzotelluropyrone intermediate compounds are converted to the corresponding telluropyrylium and benzotelluropyrylium compounds by the known methods for converting pyrones to pyryliums. These methods include:

(1) condensing a telluropyrone or benzotelluropyrone with a heterocyclic compound containing an active methyl substituent in a dehydrating medium, such as acetic anhydride;

(2) adding a Grignard reagent to the carbonyl of a telluropyrone or telluroflavone compound and treating the intermediate alcohol with an acid of the desired anion;

(3) adding an alkyl sulfonate, such as methylfluorosulfonate or ethylfluorosulfonate, to a telluropyrone or telluroflavone compound (See Example 2) below; or

(4) reducing a carbonyl or thione group of a telluropyrone or telluroflavone compound with a reducing agent, such as diisobutylaluminum hydride, and treating the intermediate alcohol with an acid of the desired anion. (This method is used in Example 1 to prepare Dye 2 in Table II, below).

The telluropyrylium dyes, including benzotelluropyrylium dyes, obtained according to the foregoing procedures are easily converted to other telluropyrylium dyes using known methods, such as:

(1) condensing a telluropyrylium or a benzotelluropyrylium compound containing an active methyl substituent with a 1,1-dialdehyde equivalent (e.g., ethyl orthoformate), 1,3-dialdehyde equivalent (e.g., 1,3,3-trimethoxypropene), or a 2-ene-1,5-dialdehyde equivalent (e.g., glutocondialdehyde dianil hydrochloride) in a dehydrating medium, such as acetic acid-acetic anhydride, in the presence of a base such as pyridine or sodium acetate (See Example 5 below);

(2) condensing a telluropyrylium compound containing an active methyl substituent with an aldehyde or a ketone in a dehydrating medium, such as acetic anhydride; or

(3) forming and decomposing a Meldrum's acid (2,2-dimethyl-m-dioxane-4,6-dione) adduct of a telluropyrone or telluroflavone compound with an acid (See Example 3 below).

Tables I, II and III below disclose dyes made according to one or more of the above procedures. The structural formula of each compound was confirmed by NMR analysis, infrared spectral analysis, mass spectral analysis or elemental analysis.

TABLE I

Telluropyrylium dyes having the structural formulae:

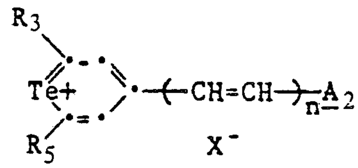

and

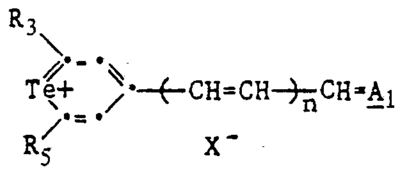

| Dye No. | $=\underline{A}_1$ or $-\underline{A}_2$ | $R_3$ | $R_5$ | n | $X^-$ | mp °C |
|---|---|---|---|---|---|---|
| 1 | $p$-$Me_2N—C_6H_4—$ | $C_6H_5$ | $C_6H_5$ | 0 | $ClO_4$ | 200—202 |
| 2 | $p$-$Me_2N—C_6H_4—$ | $C_6H_5$ | $C_6H_5$ | 0 | $BF_4$ | 192—193 |
| 3 | $p$-$Me_2N—C_6H_4—$ | $C_6H_5$ | $C_6H_5$ | 0 | $CF_3SO_2$ | 181—183 |
| 4 | 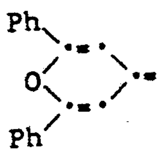 | $C_6H_5$ | $C_6H_5$ | 1 | $BF_4$ | 269—271 |
| 5 | 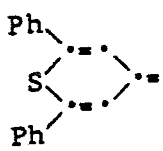 | $C_6H_5$ | $C_6H_5$ | 1 | $ClO_4$ | 213—215 |
| 6 | 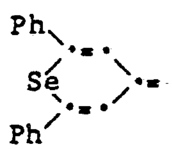 | $C_6H_5$ | $C_6H_5$ | 1 | $ClO_4$ | 208—209 |
| 7 | $CH_3CH_2O$ | $C_6H_5$ | $C_6H_5$ | 0 | $FSO_3$ | 208—210 |
| 8 | $CH_3—$ | $C_6H_5$ | $C_6H_5$ | 0 | $BF_4$ | 187—192 |
| 9 | 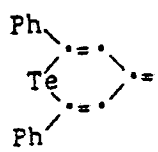 | $C_6H_5$ | $C_6H_5$ | 1 | $BF_4$ | 225—227 |
| 10 | 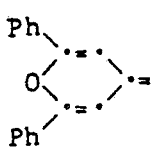 | $C_6H_5$ | $C_6H_5$ | 0 | $BF_4$ | 269—271 |
| 11 | 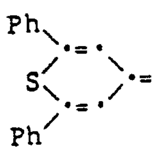 | $C_6H_5$ | $C_6H_5$ | 0 | $ClO_4$ | 213—215 |

TABLE I (continued)

| Dye No. | $=\underline{A}_1$ or $-\underline{A}_2$ | $R_3$ | $R_5$ | n | $X^-$ | mp °C |
|---|---|---|---|---|---|---|
| 12 | 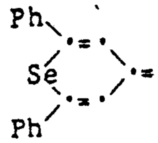 | $C_6H_5$ | $C_6H_5$ | 0 | $ClO_4$ | 208—209 |
| 13 | 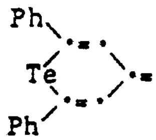 | $C_6H_5$ | $C_6H_5$ | 0 | $BF_4$ | 225—227 |
| 14 | p-$Me_2N—C_6H_4—$ | $C_6H_5$ | $C_6H_5$ | 1 | $BF_4$ | 248—250 |
| 15 | p-$Me_2N—C_6H_4—$ | $C_6H_5$ | $C_6H_5$ | 2 | $BF_4$ | |
| 16 | 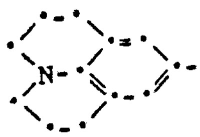 | $C_6H_5$ | $C_6H_5$ | 1 | $CF_3SO_3$ | |
| 17 | 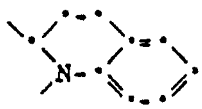 | $C_6H_5$ | $C_6H_5$ | 1 | $CF_3SO_3$ | (foam) |
| 18 | p-$Me_2N—C_6H_4—$ | $CH_3$ | $CH_3$ | O | $BF_4$ | |
| 19 | 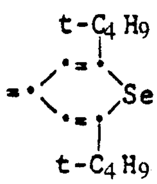 | t-$C_4H_9$ | t-$C_4H_9$ | 1 | $BF_4$ | 199—202 |

*Analysis:*
Calcd.— C (50.8); H (6.3); Te (18.6); Se (11.5); F (11.1)
Found— C (51.1); H (6.8); Te (16.9); Se (11.3); F (10.9)

| Dye No. | $=\underline{A}_1$ or $-\underline{A}_2$ | $R_3$ | $R_5$ | n | $X^-$ | mp °C |
|---|---|---|---|---|---|---|
| 20 | 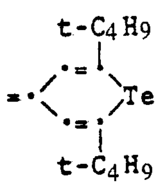 | t-$C_4H_9$ | t-$C_4H_9$ | 1 | $BF_4$ | 205—208 |

*Analysis:*
Calcd.— C (47.5); H (5.9); Te (34.8); F (10.3)
Found— C (47.4); H (6.2); Te (31.9); F (8.7)

| Dye No. | $=\underline{A}_1$ or $-\underline{A}_2$ | $R_3$ | $R_5$ | n | $X^-$ | mp °C |
|---|---|---|---|---|---|---|
| 21 | 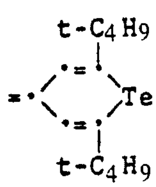 | t-$C_4H_9$ | t-$C_4H_9$ | 0 | $BF_4$ | 239—240 |
| 22 | p-$Me_2N—C_6H_4—$ | t-$C_4H_9$ | t-$C_4H_9$ | 0 | $ClO_4$ | 195—197 |

*Analysis:*
Calcd.— C (48.2); H (5.8); N (2.7); Te (24.4)
Found— C (47.8); H (5.7); N (2.6); Te (25.1)

TABLE 1 (continued)

The following three telluropyrylium dye compounds contain the $-(CH=CH)_n-CR=\underline{A}_1$ substituent which comprises the R groups noted below:

| Dye No. $=\underline{A}_1$ | | R | $R_3$ | $R_5$ | n | $X^-$ | mp °c |
|---|---|---|---|---|---|---|---|
| 23 | 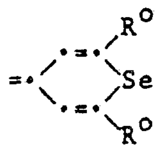 | $CH_3$ | t-$C_4H_9$ | t-$C_4H_9$ | 1 | $BF_4$ | 214.5—215.5 |

*Analysis:*
Calcd.— C (51.5); H (6.5); Te (18.3); F (10.9)
Found— C (51.8); H (6.6); Te (17.5); F (11.0)

| Dye No. $=\underline{A}_1$ | | R | $R_3$ | $R_5$ | n | $X^-$ | mp °c |
|---|---|---|---|---|---|---|---|
| 24 | 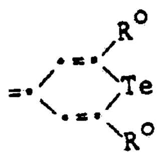 | $CH_3$ | t-$C_4H_9$ | t-$C_4H_9$ | 1 | $BF_4$ | 200—202 |

*Analysis:*
Calcd.— C (48.2); H (6.1); Te (34.1); F (10.2)
Found— C (48.0); H (6.1); Te (33.1); F (8.0)

| Dye No. $=\underline{A}_1$ | | R | $R_3$ | $R_5$ | n | $X^-$ | mp °c |
|---|---|---|---|---|---|---|---|
| 25 | 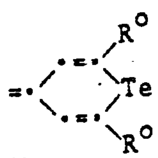 | CN | t-$C_4H_9$ | t-$C_4H_9$ | 1 | $BF_4$ | 180—185 (dec) |

$R^o$=t-$C_4H_9$

TABLE II

Benzotelluropyrylium dyes having the structural formulae:

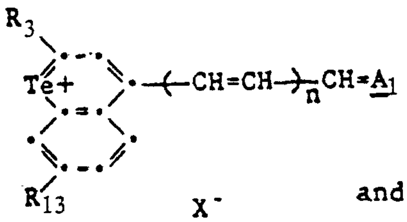

and

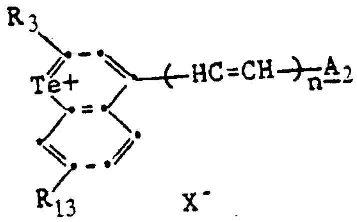

| Dye No. | $=\underline{A}_1$ or $-\underline{A}_2$ | $R_3$ | $R_{13}$ | n | $X^-$ | mp °C |
|---|---|---|---|---|---|---|
| 1 | H | $C_6H_5$ | $CH_3O$ | 0 | $ClO_4$ | 180 (dec) |
| 2 | H | $C_6H_5$ | $CH_3O$ | 0 | $PF_6$ | 160 |
| 3 | $CH_3$— | $C_6H_5$ | $CH_3O$ | 0 | $CF_3SO_3$ | 180—182 |
| 4 | $CH_3$— | $C_6H_5$ | $CH_3O$ | 0 | $BF_4$ | 182—190 (dec) |
| 5 | $CH_3$— | $C_6H_5$ | $CH_3O$ | 0 | $ClO_4$ | 180 (dec) |
| 6 | $CH_3O$— | $C_6H_5$ | $CH_3O$ | 0 | $ClO_4$ | 180 (dec) |
| 7 | $CH_3CH_2O$ | $C_6H_5$ | $CH_3O$ | 0 | $FSO_3$ | 215—216 |
| 8 | $CH_3CH_2O$ | $CH_3$ | $CH_3O$ | 0 | $FSO_3$ | 50 (dec) |
| 9 | *p*-$Me_2N$—$C_6H_4$— | $C_6H_5$ | $CH_3O$ | 0 | $BF_4$ | 142—152 (dec) |
| 10 | *p*-$Me_2N$—$C_6H_4$— | $C_6H_5$ | $CH_3O$ | 0 | $ClO_4$ | 160 (dec) |
| 11 | *p*-$Me_2N$—$C_6H_4$— | $C_6H_5$ | $CH_3O$ | 1 | $CF_3SO_3$ | 200—204 |
| 12 | *p*-$Me_2N$—$C_6H_4$— | $C_6H_5$ | $CH_3O$ | 1 | $BF_4$ | 225—227 |
| 13 | *p*-$Me_2N$—$C_6H_4$— | $C_6H_5$ | $CH_3O$ | 2 | $CF_3SO_3$ | 240 (dec) |
| 14 | $Me_2N$— | $C_6H_5$ | $CH_3O$ | 1 | $CF_3SO_3$ | 194—196 |
| 15 | 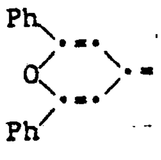 | $C_6H_5$ | $CH_3O$ | 0 | $BF_4$ | 140—145 |
| 16 | 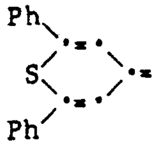 | $C_6H_5$ | $CH_3O$ | 0 | $ClO_4$ | 135—142 |

TABLE II (continued)

| Dye No. | $=\underline{A}_1$ or $-\underline{A}_2$ | $R_3$ | $R_{13}$ | n | $X^-$ | mp °C |
|---|---|---|---|---|---|---|
| 17 | 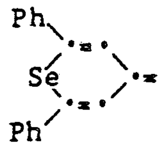 | $C_6H_5$ | $CH_3O$ | 0 | $ClO_4$ | 140 (dec) |
| 18 | 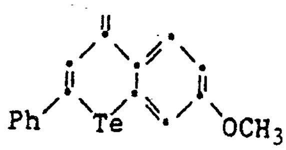 | $C_6H_5$ | $CH_3O$ | 1 | $CF_3SO_3$ | 200 (dec) |
| 19 | 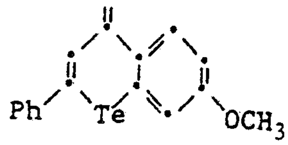 | $C_6H_5$ | $CH_3O$ | 1 | $ClO_4$ | |
| 20 | 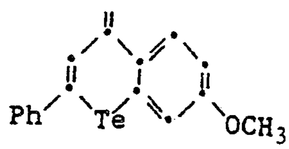 | $C_6H_5$ | $CH_3O$ | 1 | $PF_6$ | |
| 21 | 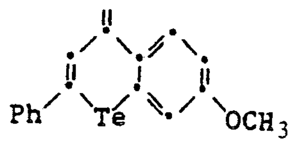 | $C_6H_5$ | $CH_3O$ | 1 | $CF_3SO_3$ | 200 (dec) |
| 22 | 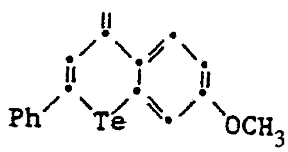 | $C_6H_5$ | $CH_3O$ | 2 | $PF_6$ | |
| 23 | 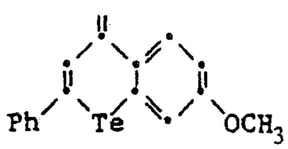 | $C_6H_5$ | $CH_3O$ | 2 | $CF_3SO_3$ | 215—219 |
| 24 | 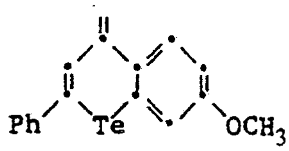 | $C_6H_5$ | $CH_3O$ | 3 | $CF_3SO_3$ | |
| 25 | 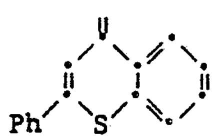 | $C_6H_5$ | $CH_3O$ | 1 | $CF_3SO_3$ | 225—226 |
| 26 | 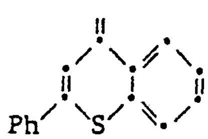 | $C_6H_5$ | $CH_3O$ | 2 | $CF_3SO_3$ | 230—231 |

TABLE II (continued)

| Dye No. | $=\underline{A}_1$ or $-\underline{A}_2$ | $R_3$ | $R_{13}$ | n | $X^-$ | mp °C |
|---|---|---|---|---|---|---|
| 27 | 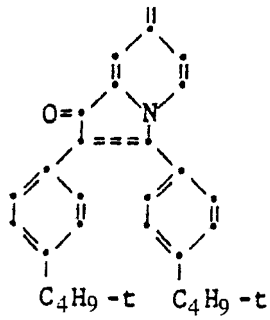 | $C_6H_5$ | $CH_3O$ | 1 | $BF_4$ | |
| 28 | 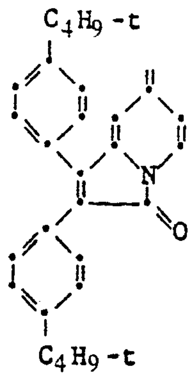 | $C_6H_5$ | $CH_3O$ | 1 | $Bf_4$ | |
| 29 | 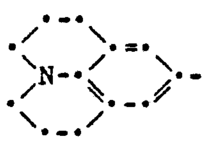 | $C_6H_5$ | $CH_3O$ | 1 | $PF_6$ | |
| 30 | $C_6H_5$— | $CH_3$ | $CH_3O$ | 0 | $CF_3SO_3$ | 153—163 |
| 31 | $C_6H_5$— | *p*-$Me_2N$—$C_6H_4$— | $CH_3O$ | 1[a] | $CF_3SO_3$ | |
| 32 | *p*-$Me_2N$—$C_6H_4$— | $CH_3CH_2O$ | $CH_3O$ | 1[a] | $FSO_3$ | 163—167 |
| 33 | *p*-$Me_2N$—$C_6H_4$— | $CH_3CH_2O$ | $CH_3O$ | 2[a] | $FSO_3$ | 175—178 |
| 34 | 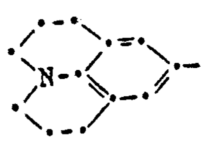 | $CH_3CH_2O$ | $CH_3O$ | 1[a] | $FSO_3$ | 175—179 |
| 35 | 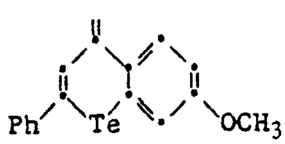 | $CH_3CH_2O$ | $CH_3O$ | 0[a] | $FSO_3$ | 204 (dec) |

[a]=these compounds refer to the following structural formulae:

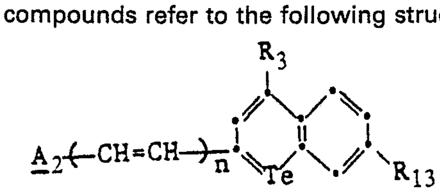

and

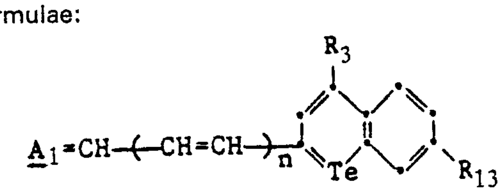

TABLE III

This Table illustrates telluropyrylium dyes having zwitterionic diketonate substituents. In the table, Ph represents phenyl.

| | | mp °C |
|---|---|---|
| 1 | 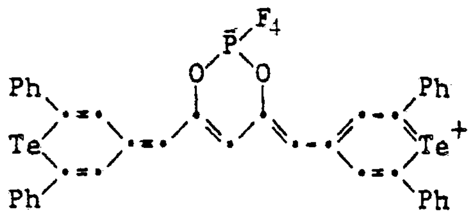 | 201-204(dec) |
| 2 | 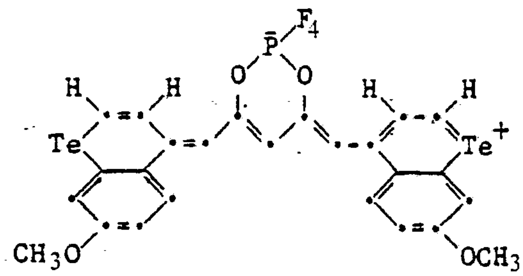 | 230-245(dec) |
| 3 | 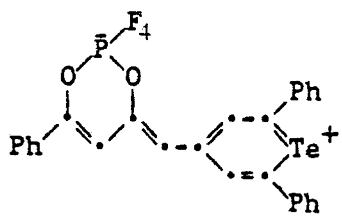 | 119-122(dec) |
| 4 | 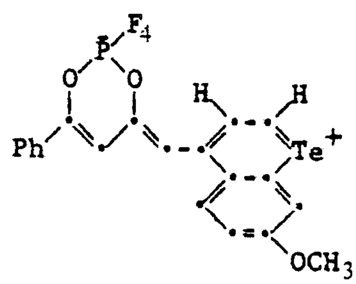 | 185(dec) |
| 5 | 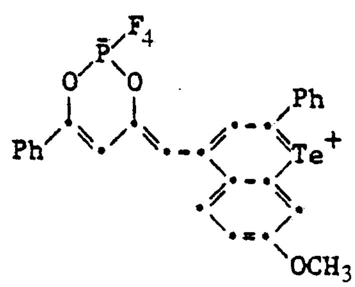 | 204-207(dec) |
| 6 | 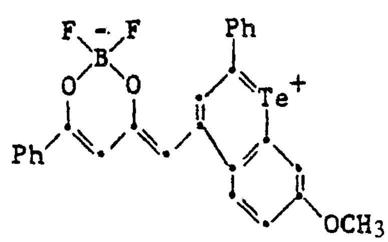 | 117-119 |

TABLE III (cont.)

7 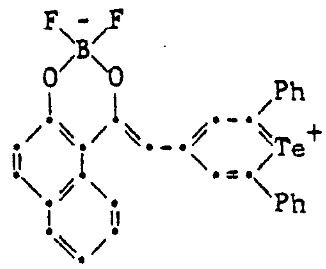 262-266

8 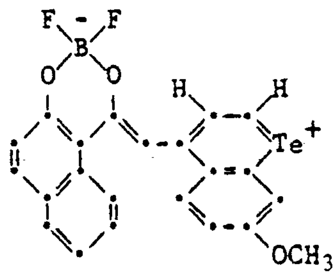 220-222

9 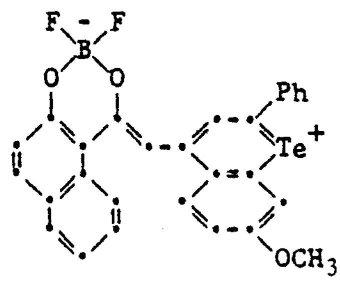 and 232-235

10 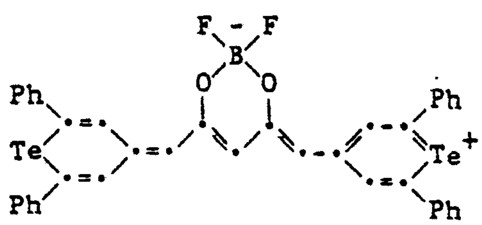 200-204

The present invention provides photoconductive compositions and in which organic electron donor-type photoconductor compounds are combined with sensitizing amounts of the electron accepting dyes of the present invention.

The compositions are prepared by blending a dispersion or solution of the donor-type photoconductor compound together with an electrically insulating, film-forming resin binder, when necessary or desirable, and coating the compositions on a support or forming a self-supporting layer with the photoconductive composition. A sensitizing amount of the dye compound is mixed with the photoconductive coating composition so that, after thorough mixing, the sensitizing dye is uniformly distributed throughout a layer formed from the composition. The amount of dye which can be added to a photoconductive composition layer to provide effective results may be as low as 0.001 to as much as 30 percent by weight based on the weight, of the film-forming coating composition. The usual amount of the dye is from 0.05 to 10 percent by weight of the coating composition.

The dyes used in this invention are effective for enhancing the photosensitivity of a wide variety of donor-type photoconductors compounds, especially those containing a tertiary amine component. Useful photoconductor compounds are described below:

(1) arylamine photoconductor compounds, including substituted and unsubstituted arylamines, diarylamines, non-polymeric triarylamines and polymeric triarylamines such as those described in U.S. Patents 3,240,597 and 3,180,730;

(2) polyarylalkane photoconductor compounds of the types described in U.S. Patents 3,274,000; 3,542,547; and 3,542,544;

(3) 4-diarylamino-substituted chalcone compounds of the types described in U.S. Patent 3,526,501;

(4) nonionic cycloheptenyl compounds of the types described in U.S. Patent 3,533,786;

(5) compounds containing an

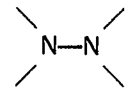

nucleus, as described in U.S. Patent 3,542,546;

(6) organic compounds having a 3,3′-bisaryl-2-pyrazoline nucleus, as described in U.S. Patent 3,527,602;

(7) triarylamine compounds in which at least one of the aryl radicals is substituted by either a vinyl radical or a vinylene radical having at least one active hydrogen-containing group, as described in U.S. Patent 3,567,450;

(8) triarylamine compounds in which at least one of the aryl radicals is substituted by an active hydrogen-containing group, as described in Belgian Patent 728,563;

(9) any other organic donor compound which exhibits photoconductive properties, such as those set forth in Australian Patent 248,402, and the various polymeric photoductor compounds such as the carbazol polymers described in U.S. Patent 3,421,891.

The photoconductive compositions of the present invention can be employed in any of the well-known electrophotographic processes which require photoconductive layers.

The following examples are presented to further illustrate this invention.

## Example 1

Preparation of Dye 2, Table II (7-Methoxy-2-phenylbenzo[b]telluropyrylium Hexafluorophosphate)

Ten ml of a 22% w/w solution of diisobutylaluminum hydride (w/w) in hexane were added to a slurry of 1.81 g of 7-methoxy-2-phenylbenzotelluropyrone in 20 ml of toluene cooled to 0°C. The cooling bath was removed and the reaction mixture was warmed to ambient temperature. Twenty ml of 1N sodium hydroxide were then added. The resulting mixture was stirred for 1 hour at ambient temperature and was then diluted with 100 ml of ether. The organic phase was washed with sodium chloride solution and concentrated. The residue was dissolved in 25 ml of acetic acid and 2 ml of hexafluorophosphoric acid was added. Upon cooling, a red solid precipitated. The precipitate was filtered, washed with ether and dried.

## Example 2

Preparation of Dye 7, Table II (4-Ethoxy-2-phenyl-7-methoxybenzo[b]telluropyrylium Fluorosulfonate)

Five g of 7-methoxy-2-phenylbenzotelluropyrone were added to 20 ml of freshly distilled ethyl fluorosulfonate. The resulting mixture was stirred under nitrogen at 60°C for 10 minutes. Two hundred ml of ether were then added, precipitating a red solid. The product was collected by filtration and recrystallized from acetonitrile to yield 6.0 g (87%) of a red solid.

## Example 3

Preparation of Dye 3, Table II (4-Methyl-7-methoxybenzo[b]telluropyrylium Trifluoromethanesulfonate)

Meldrum's acid (2,2-dimethyl-m-dioxane-4,6-dione) (0.29 g) was dissolved in 10 ml of pyridine. The 4-ethoxy-7-methoxybenzotelluropyrylium fluorosulfonate (1.0 g, 2.0 mmol) was added as a powder immediately giving a dark red solution. The solution was concentrated *in vacuo*. The residue was purified by chromatography on silica gel eluting with methylene chloride to yield 0.90 g (92%) of a red solid.

The red solid (22.0 g, 0.0449 mol) was dissolved in 440 ml of 97% formic acid. The reaction mixture was stirred with steam bath heating until gas evolution ceased. Trifluoromethane sulfonic acid (8.4 g, 0.056 mol) was added and the reaction mixture was concentrated *in vacuo*. Ether (400 ml) was added precipitating 18.4 g (80%) of a red solid. The crude product was dissolved in acetonitrile and precipitated with ether to yield 13.8 g (60%) of material.

## Example 4

Preparation of Dye 1, Table I (4-(p-N,N-Dimethylaminophenyl)-2,6-diphenyltelluropyrylium Perchlorate

A solution of *p*-bromo-N,N-dimethylaniline (1.00 g) in 5 ml of dry tetrahydrofuran was added to magnesium turnings (0.24 g) under an argon atmosphere. A small crystal of iodine was added and the resulting mixture was warmed at reflux for 2 hours. 2,6-Diphenyltelluropyrone (0.20 g) in 5 ml of dry tetrahydrofuran was added dropwise. The resulting mixture was stirred at reflux for an additional hour. The tetrahydrofuran solution was decanted from the magnesium turnings and concentrated. The residue was dissolved up in 5 ml of acetic acid. One ml of 70% perchloric acid was added. The resulting solution was added dropwise into 20 ml of cold water. The precipitate was collected by filtration and recrystallized from acetonitrile to yield 0.26 g (81%) of a copper-bronze colored solid.

## Example 5

Preparation of Dye 11, Table I 4-(2,6-Diphenyl-4-thiapyranylidenemethyl)-2,6-diphenyltelluropyrylium Perchlorate

2,6-Diphenyltelluropyrone (0.50 g, 1.4 mmol) and 4-methyl-2,6-diphenylthiapyrylium perchlorate (0.50 g, 1.4 mmol) in 5 ml of acetic anhydride were warmed on a steam bath for 15 minutes and then chilled. The

dye was collected by filtration and recrystallized from acetonitrile to yield 0.85 g (88%) of copper-colored needles.

Example 6

Preparation of Dye 12, Table II 4-(p-N,N-Dimethylaminostyryl)-7-methoxy-2-phenylbenzo[b]telluropyrylium Tetrafluoroborate

N,N-Dimethylaminobenzaldehyde (0.50 g, 3.3 mmol) and 4-methyl-7-methoxy-2-phenylbenzotelluropyrylium tetrafluoroborate (0.60 g, 1.3 mmol) in 5 ml of acetic anhydride were warmed on a steam bath for 3 minutes. Acetonitrile (10 ml) was added and the reaction mixture was chilled. The crude product was collected by filtration and recrystallized from acetonitrile to yield 0.34 g (44%) of a golden-brown colored solid.

Example 7

Preparation of Dye 23, Table II

A mixture of 1,3,3-trimethoxypropene (0.5 ml) and 4-methyl-7-methoxy-2-phenylbenzotelluropyrylium trifluoromethanesulfonate (0.55 g, 1.0 mmol) in 2 ml of acetic anhydride, 1.5 ml of acetic acid, and 0.5 ml of pyridine was warmed on a steam bath for 1.5 minutes and was then chilled. The crude product was collected by filtration and recrystallized from acetonitrile to yield 0.22 g (49%) of a brown colored solid.

Example 8

Preparation of 4-(2,6-Diphenyltelluropyranylidene)benzoylacetonatophosphorous Tetrafluoride

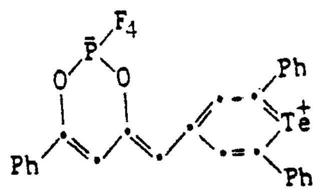

Benzoylacetonatophosphorous tetrafluoride (0.27 g 1.0 mmol) and 2,6-diphenyltelluropyrone (0.38 g, 1.1 mmol) in 3 mL of acetic anhydride were warmed on a steam bath for 20 minutes. Acetonitrile (3 ml) was then added and the resulting mixture was chilled. The product was collected by filtration and recrystallized from acetonitrile to yield 0.17 g (28%) of a metallic green solid.

Example 9

The following examples show the use of dyes of the present invention as sensitizers in electrophotographic elements. Each film was formulated and coated as follows. Ten to fifteen mg of a dye from Tables I, II or III plus 215 to 300 mg of tri-*p*-tolylamine were dissolved in 3 ml of dichloromethane. To this solution were added 4 ml of dichloromethane containing 12.5% by weight of Lexan-145TM (a bisphenol polycarbonate available from General Electric Co.). The solution was stirred for several minutes and then coated at .006 mil wet thickness on a poly(ethylene terephthalate) support containing 0.4 OD evaporated nickel. After initial evaporation of the solvent, the films were dried for 24 hours in air at 60°C. Dry thickness was 7 µm.

The quantum efficiency ($\phi_o$) of each film was measured as follows. Samples were corona-charged to a surface potential equivalent to the field strengths, $E_o$, indicated in Table IV. They were then exposed to monochromatic radiation at the wavelength indicated in Table IV (λ, nm) with a bandwidth of 10 nm. The incident photon flux at this wavelength was measured with an Optronics Laboratories Model 730-A Radiometer. Films were allowed to discharge while exposed to the indicated radiation. The initial quantum efficiency (the number of electron-hole pairs produced per incident photon) at field strength $E_o$ was then determined by computation of the slope of the discharge curve at $E_o$. The photodischarge sensitivity at wavelength of irradiation ($S_{1/2}$), was also determined by allowing the films to discharge from $E_o$ to $E_o/2$. The amount of radiation necessary to produce this discharge was then calculated from the time required for this half-decay and the incident photon flux.

Dyes 1, 10, 11, 12 and 13 of Table I, Dye 10 of Table II and Dyes 1, 3, 5, 7, 8 and 9 of Table III were tested as described above. Each of the dyes resulted in an increase in the speed and/or quantum efficiency of the photoconductive layers in which they were included. The data from the tests are presented in Table IV.

TABLE IV
Telluropyrylium dyes as Sensitizers for Tri-p-tolylamine Photoconductors

| Film No. | Sensitizer | λ nm | $E_o$ V/cm | $\Phi_o$ | $S_{1/2}$ ergs/cm² |
|---|---|---|---|---|---|
| 1 | None | 350 | $1.6 \times 10^6$ | 0.0094 | 1500 |
| 2 | No. 1, Table I | 760 | $3.3 \times 10^5$ | 0.036 | 58 |
| 3 | No. 10, Table I | 630<br>670 | $6.1 \times 10^5$<br>$4.9 \times 10^5$ | 0.0063<br>0.0044 | 315<br>271 |
| 4 | No. 11, Table I | 660<br>715 | $6.2 \times 10^5$<br>$4.5 \times 10^5$ | 0.008<br>0.0052 | 135<br>169 |
| 5 | No. 12, Table I | 730 | $3.1 \times 10^5$ | 0.0024 | 424 |
| 6 | No. 13, Table I | 780 | $4.4 \times 10^5$ | 0.0015 | 823 |
| 7 | No. 10, Table II | 660 | $5.7 \times 10^5$ | 0.011 | 193 |
| 8 | No. 7, Table III | 625 | $6.2 \times 10^5$ | 0.0042 | 430 |
| 9 | No. 1 Table III | 800 | — | — | 1242 |
| 10 | No. 3 Table III | 605 | $5.8 \times 10^5$ | 0.0046 | 202 |
| 11 | No. 5 Table III | 615 | $1.5 \times 10^6$ | 0.14 | 44 |
| 12 | No. 8 Table III | 595 | $5.6 \times 10^5$ | 0.0042 | 557 |
| 13 | No. 9 Table III | 620 | $1.2 \times 10^6$ | 0.051 | 84 |

Film number 1 consisted of 30% by weight of tri-*p*-tolylamine. Film numbers of 2, 3, 4, 5, 6, 7, 8, 9, 11 and 13 consisted by weight of 1.5% of a dye of the invention, as indicated, 38.5% tri-*p*-tolylamine and 60% Lexan 145. Film number 7 consisted by weight of 2% dye, 30% tri-*p*-tolylamine and 68% Lexan 145. Film number 10 consisted by weight of 2% dye, 38% tri-*p*-tolylamine and 60% Lexan 145. Film number 12 consisted by weight of 1% dye, 39% tri-*p*-tolylamine and 60% Lexan 145.

**Claims**

1. A telluropyrylium compound having the structural formula

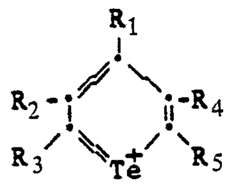

$(X^-)_m$; I.

wherein

$R_1$, $R_3$ and $R_5$ each independently represent a hydrogen atom or alkyl, alkylamino, dialkylamino, alkoxy, alkylaryl, aryl or aryloxy group where each alkyl or alkoxy is a substituted or unsubstituted, branched or straight-chain hydrocarbon having from 1 to 16 carbon atoms; and each aryl or aryloxy is a substituted or unsubstituted phenyl, naphthyl or anthryl group; or a heterocyclic group having from 5 to 20 carbon atoms where the hetero atoms are oxygen, phosphorus, nitrogen, sulfur, selenium or tellurium; or a

$-(CH{=}CH)_n-CH{=}\underline{A}_1$ or $-(CH{=}CH)_n-\underline{A}_2$ group;

or a zwitterionic diketonate of the structural formula:

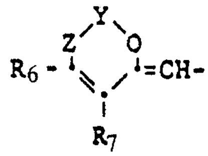 ;

$R_2$ and $R_4$ each independently represent a hydrogen atom or a substituted or unsubstituted, branched or straight-chain alkyl group having from 1 to 16 carbon atoms; or a substituted or an unsubstituted phenyl, naphthyl, or anthryl group; or

$R_2$ and $R_3$, or $R_4$ and $R_5$, taken together with the carbon atoms to which they are attached, form a fused mono- or polynuclear carbocyclic ring system having from 5 to 20 carbon atoms;

$R_6$ and $R_7$ each independently represent a hydrogen atom or a substituted or unsubstituted, branched or straight-chain alkyl group having from 1 to 16 carbon atoms; or a substituted or unsubstituted phenyl, naphthyl or anthryl group; or a substituted or unsubstituted heterocyclic group where the hetero atom is oxygen, phosphorus, nitrogen, sulfur, selenium or tellurium; or a mono- or polycyclic heterocyclylidene methyl group wherein the hetero atom is oxygen, phosphorus, nitrogen, sulfur, selenium or tellurium; or $R_6$ and $R_7$, taken together with the carbon atoms to which they are attached, form a fused mono- or polycyclic, carbocyclic or heterocyclic group having from 5 to 20 carbons wherein the hetero atoms are oxygen, phosphorus, nitrogen, sulfur, selenium or tellurium;

$\underline{A}_1$ represents a mono- or polycyclic heterocyclic group conjugated through the methine or the polymethine linking group, to the telluropyrylium ring wherein the hetero atoms are oxygen, phosphorus, nitrogen, sulfur, selenium or tellurium and the cyclic ring system has from 5 to 20 carbon atoms;

$\underline{A}_2$ represents a hydrogen atom, amino, alkyl, alkylamino, dialkylamino, dialkylaminoaryl or alkoxy group where each alkyl or alkoxy is a substituted or unsubstitued, branched or straight-chain hydrocarbon having from 1 to 16 carbon atoms; or substituted or unsubstituted aryl, arylamino or diarylamino group where each aryl is a phenyl, naphthyl or anthryl group; or a mono or polycyclic heterocyclic group having from 5 to 20 carbon atoms where the hetero atom is oxygen, phosphorus, nitrogen, sulfur, selenium or tellurium, said heterocyclic group being conjugated, directly or indirectly through the polymethine linking group, to the telluropyrylium ring;

n is a number from 0 to 5;

m is 1, except when $R_1$, $R_3$ or $R_5$ is a zwitterionic group, then m is 0;

X is an anion;

Y is $BF_2$, or $PF_4$; and

Z is an oxygen or sulfur atom.

2. A compound according to Claim 1 wherein $R_1$, $R_3$ and $R_5$ each independently represents a methyl, ethyl, phenyl, p-N,N-dimethylaminophenyl, p-anisyl, phenoxy, ethoxy, methoxy; or a

$$-(CH=CH)_n-CH=\underline{A}_1 \text{ or } -(CH=CH)_n-\underline{A}_2 \text{ group;}$$

or a zwitterionic diketonate of the structural formula:

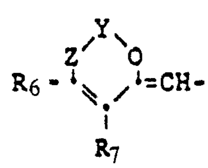 ;

$R_6$ and $R_7$ each independently represents a methyl, phenyl, methoxyphenyl, p-N,N-dimethylaminophenyl, aminophenyl, pyridyl, oxaxolyl, thiazolyl, selenazolyl, pyranyl, thiapyranyl, selenapyranyl, telluropyranyl, or oxaindolazinyl group; or $R_6$ and $R_7$, taken together with the carbon atoms to which they are attached, are as defined in claim 1;

$\underline{A}_1$ represents an oxazolylidene, thiazolylidene, selenazolylidene, imidazolylidene, pyranylidene, thiapyranylidene, selenapyranylidene, telluropyranylidene, oxaindolazinylidene, benzoxazolylidene, benzothiazolylidene, benzopyranylidene, benzothiapyranylidene, benzoselenapyranylidene, or benzotelluropyranylidene nucleus;

$\underline{A}_2$ represents a hydrogen atom or a methyl, methoxy, ethoxy, phenyl, dimethylaminophenyl or dimethylamino group, or an oxazolyl, 9-julolidyl, pyridyl, thiazolyl, selenazolyl, imidazolyl, pyryliumyl, thiapyryliumyl, selenapyrylium, telluropyrylium, pyridinyl, furanyl, thiophenyl, selenophenyl, tellurophenyl, oxaindolazinyl, benzoxazolyl, benzothiazolyl, benzopyryliumyl, benzothiapyryliumyl, benzoselenapyryliumyl, benzotelluropyryliumyl or naphthyl nucleus; and

X represents $BF_4$, $ClO_4$, $SO_3$, $CF_3$, $CF_3SO_3$, $FSO_3$, $PF_6$, $CH_3SO_2$, chlorine, bromine or iodine.

3. A compound according to claim 1 wherein $R_2$ and $R_3$ or $R_4$ and $R_5$, taken together with the carbon atoms to which they are attached, form a benzene nucleus.

4. A compound according to claim 3, wherein the benzotelluropyrylium nucleus has the structural formula:

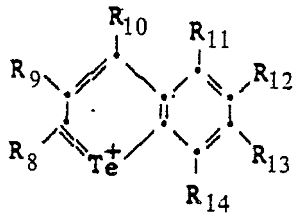

III.

$(X^-)_{m'}$;

wherein,

$R_8$ and $R_{10}$ each independently represent a hydrogen atom or an alkyl, alkylamino, arylamino, alkoxy, aryloxy, dialkylamino, diarylamino,

$$-(CH=CH)_{n'}-CH=\underline{A}_1 \text{ or } -(CH=CH)_{n'}-\underline{A}_2 \text{ group;}$$

$R_9$ represents a hydrogen atom or a substituted or unsubstituted, branched or straight-chain alkyl group having from 1 to 16 carbon atoms; or a substituted or an unsubstituted phenyl, naphthyl or anthryl group;

$\underline{A}_1$ and $\underline{A}_2$ are as defined in claim 1;

$R_{11}$, $R_{12}$, $R_{13}$ and $R_{14}$ each independently represent a hydrogen or a halogen atom or an alkyl, alkoxy, aryl, alkylamino or arylamino group; or

$R_{11}$ and $R_{12}$, $R_{12}$ and $R_{13}$ or $R_{13}$ and $R_{14}$, taken together with the carbon atoms to which they are attached, form a fused carbocyclic ring system having from 5 to 16 carbon atoms;

n' is 0, 1 or 2;

m' is 1

5. A compound according to Claim 4 wherein

$R_8$ and $R_{10}$ each independently represents a hydrogen atom or a methyl, ethyl, methoxy, hydroxy, ethoxy, phenyl, phenoxy, p-anisyl, 2,5-di-methoxy-phenyl, p-N,N-dimethylaminophenyl, diarylamino,

$$-(CH=CH)_{n'}-CH=\underline{A}_1 \text{ or } -(CH=CH)_{n'}-\underline{A}_2 \text{ group;}$$

$\underline{A}_1$ and $\underline{A}_2$ are as defined in claim 2;

X represents $BF_4$, $ClO_4$, $CF_3SO_3$, $FSO_3$, $PF_6$, $CH_3SO_2$, chlorine, bromine or iodine.

6. A photoconductive composition comprising an electron donating organic photoconductor and a sensitizing agent of a telluropyrylium compound as defined in Claim 1, 2, 3, 4 or 5 wherein said compound is present in an amount of from .001 to 30% based on the weight of said composition.

7. A photoconductive composition according to Claim 6 wherein the electron donating photoconductor comprises a tertiary amine, optionally a triarylamine.

**Patentansprüche**

1. Telluropyryliumverbindung der Strukturformel

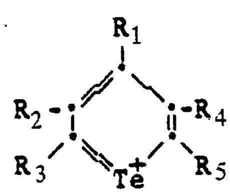

I.

$(X^-)_m$;

worin bedeuten:

$R_1$, $R_3$ und $R_5$ jeweils unabhängig voneinander ein Wasserstoffatom oder eine Alkyl-, Alkylkamino-, Dialkylamino-, Alkoxy-, Alkylaryl-, Aryl oder Aryloxygruppe, wobei Alkyl und Alkoxy jeweils für einen substituierten oder unsubstituierten, verzweigtkettigen oder geradkettigen Kohlenwasserstoffrest mit 1 bis 16 Kohlenstoffatomen stehen und Aryl und Aryloxy jeweils für eine substituierte oder unsubstituierte Phenyl-, Naphthyl- oder Anthrylgruppe oder eine heterocyclische Gruppe mit 5 bis 20 Kohlenstoffatomen, wobei die Heteroatome Sauerstoff-, Phosphor, Stickstoff-, Schwefel-, Selen- oder Telluratome sein können; oder eine Gruppe einer der folgenden Formeln

$$-(CH=CH)_n-CH=\underline{A}_1 \text{ oder } -(CH=CH)_n-\underline{A}_2$$

oder zwitterionisches Diketonat der Strukturformel:

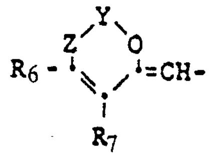
;

$R_2$ und $R_4$ jeweils unabhängig voneinander ein Wasserstoffatom oder eine substituierte oder unsubstituierte, verzweigtkettige oder geradkettige Alkylgruppe mit 1 bis 16 Kohlenstoffatomen oder eine substituierte oder unsubstituierte Phenyl-, Naphthyl- oder Anthrylgruppe; oder

$R_2$ und $R_3$ oder $R_4$ und $R_5$ gemeinsam mit dem Kohlenstoffatom, an das sie gebunden sind, ein ankondensiertes mono- oder polynukleares carbocyclisches Ringsystem mit 5 bis 20 Kohlenstoffatomen;

$R_6$ und $R_7$ jeweils unabhängig voneinader ein Wasserstoffatom oder eine substituierte oder unsubstituierte, verzweigtkettige oder geradkettige Alkylgruppe mit 1 bis 16 Kohlenstoffatomen; oder eine substituierte oder unsubstituierte Phenyl-, Naphthyl-, oder Anthrylgruppe oder eine substituierte oder unsubstituierte heterocyclische Gruppe, in der das Heteroatom ein Sauerstoff-, Phosphor-, Stickstoff-, Schwefel-, Selen- oder Telluratom ist; oder eine mono- oder polycyclische Heterocyclidenmethylgruppe, in der das Heteroatom ein Sauerstoff-, Phosphor-, Stickstoff-, Schwefel-, Selen- oder Telluratom ist oder.

$R_6$ und $R_7$ gemeinsam mit dem Kohlenstoffatom, an das sie gebunden sind, eine ankondensierte mono- oder polylcyclische, carbocyclische oder heterocyclische Gruppe mit 5 bis 20 Kohlenstoffatomen, in der das Heteroatom ein Sauerstoff-, Phosphor-, Stickstoff-, Schwefel-, Selen- oder Telluratom ist;

$\underline{A}_1$ eine mono- oder polycyclische heterocyclische Gruppe, die durch die $\underline{A}_1$ mit dem Telluropyryliumring verbundene Methin- oder Polymethingruppe konjugiert ist, wobei in dem Telluropyryliumring die Heteroatome Sauerstoff-, Phosphor-, Stickstoff-, Schwefel-, Selen- oder Telluratome sind und das cyclische Ringsystem 5 bis 20 Kohlenstoffatome aufweist;

$\underline{A}_2$ ein Wasserstoffatom, eine Amino-, Alkyl-, Alkylamino-, Dialkylamino-, Dialkylaminoaryl- oder Alkoxygruppe, wobei gilt, daß jede Alkyl- oder Alkoxygruppe eine substituierte oder unsubstituierte, verzweigtkettige oder geradkettige Kohlenwasserstoffgruppe mit 1 bis 16 Kohlenstoffatomen ist; oder eine substituierte oder unsubstituierte Aryl-, Arylamino- oder Diarylaminogruppe, in der jede Arylgruppe eine Phenyl-, Naphthyl- oder Anthrylgruppe ist; oder eine mono- oder polycyclische heterocyclische Gruppe mit 5 bis 20 Kohlenstoffatomen, in der das Heteratom ein Sauerstoff-, Phosphor-, Stickstoff-, Schwefel-, Selen- oder Telluratom ist, wobei die heterocyclische Gruppe direkt oder indirekt durch die verbindende Polymethingruppe an den Telluropyryliumring konjugiert ist;

n eine Zahl von 0 bis 5;

m gleich 1, mit der Ausnahme, daß m gleich 0 ist, wenn $R_1$, $R_3$ oder $R_5$ eine zwitterionische Gruppe ist;

X ein Anion;

Y gleich $BF_2$ oder $PF_4$ und

Z ein Sauerstoff- oder Schwefelatom.

2. Eine Verbindung nach Anspruch 1, dadurch gekennzeichnet, daß in der angegebenen Formel bedeuten;

$R_1$, $R_3$ und $R_5$ jeweils unabhängig voneinander eine Methyl-, Ethyl-, Phenyl-, p-N,N-Dimethylaminophenyl-, p-Anisyl-, Phenoxy-, Ethoxy- oder Methoxygruppe; oder eine Gruppe der Formeln:

$$-\!(CH=CH)_n\!-CH=\underline{A}_1 \text{ oder } -\!(CH=CH)_n\!-\underline{A}_2$$

oder eine zwitterionisches Diketonat der folgenden Strukturformel;

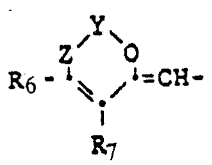
;

$R_6$ und $R_7$ jeweils unabhängig voneinander eine Methyl-, Phenyl-, Methoxyphenyl-, p-N,N-Dimethylaminophenyl-, Aminophenyl-, Pyridyl-, Oxazolyl-, Thiazolyl-, Selenazolyl-, Pyranyl-, Thiapyranyl-, Selenapyranyl-, Telluropyranyl-, oder Oxaindolazinylgruppe; oder $R_6$ und $R_7$ gemeinsam mit den Kohlenstoffatomen, an die sie gebunden sind, eine Gruppe wie in Anspruch 1 angegeben;

$\underline{A}_1$ einen Oxazolyliden-, Thiazolyliden-, Selenazolyliden-, Imidazolyliden-, Pyranyliden-, Thiapyranyliden-, Selenapyranyliden-, Telluropyranyliden, Oxaindolazinyliden-, Benzoxazolyliden-, Benzothiazolyliden-, Benzopyranyliden-, Benzothiapyranyliden-, Benzoselenapyranyliden- oder Benzotelluropyranylidenkern;

$\underline{A}_2$ ein Wasserstoffatom oder eine Methyl-, Methoxy-, Ethoxy-, Phenyl-, Dimethylaminophenyl- oder Dimethylaminogruppe oder einen Oxazolyl-, 9-Julolidyl-, Pyridyl-, Thiazolyl-, Selenazolyl-, Imidazolyl-, Pyryliumyl-, Thiapyryliumyl-, Selenapyrylium-, Telluropyrylium-, Pyridinyl-, Furanyl-, Thiophenyl-, Selenophenyl-, Tellurophenyl-, Oxaindolazinyl-, Benzoxazolyl-, Benzothiazolyl-, Benzopyryliumyl-, Benzothiapyryliumyl-, Benzoselenapyryliumyl-, Benzotelluropyryliumyl- oder Naphthylkern und

X gleich $—BF_4$, $—ClO_4$, $—SO_3$, $—CF_3$, $—CF_3SO_3$, $—FSO_3$, $—PF_6$, $—CH_3SO_3$, Chlor, Brom oder Jod.

3. Eine Verbindung nach Anspruch 1, dadurch gekennzeichnet, daß in der Formnel $R_2$ und $R_3$ oder $R_4$ und $R_5$ gemeinsam mit den Kohlenstoffatomen, an die sie gebunden sind, einen Benzolring bilden.

4. Eine Verbindung nach Anspruch 3, dadurch gekennzeichnet, daß der Benzotelluropyryliumkern der folgenden Struktur- formel entspricht:

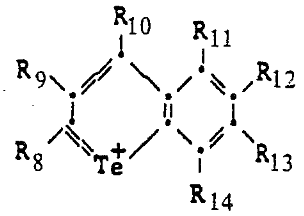

$(X^-)_{m'}$; III.

in der bedeuten:

$R_8$ und $R_{10}$ jeweils unabhängig voneinander ein Wasserstoffatom oder ein Alkyl-, Alkylamino-, Arylamino-, Alkoxy-, Aryloxy-, Dialkylamino- oder Diarylaminogruppe oder eine Gruppe der Formeln:

$$-\!(CH{=}CH)_{\overline{n'}}CH{=}\underline{A}_1 \text{ oder } -\!(CH{=}CH)_{\overline{n'}}\underline{A}_2;$$

$R_9$ ein Wasserstoffatom oder eine substituierte oder unsubstituierte, verzweigtkettige oder geradkettige Alkylgruppe mit 1 bis 16 Kohlenstoffatomen; oder eine substituierte oder eine unsubstituierte Phenyl-, Naphthyl- oder Anthrylgruppe;

$\underline{A}_1$ und $\underline{A}_2$ wie in Anspruch 1 angegeben;

$R_{11}$, $R_{12}$, $R_{13}$ und $R_{14}$ jeweils unabhängig voneinander ein Wasserstoffatom oder ein Halogenatom oder eine Alkyl-, Alkoxy-, Aryl-, Alkylamino- oder Arylaminogruppe oder

$R_{11}$ und $R_{12}$, $R_{12}$ und $R_{13}$ oder $R_{13}$ und $R_{14}$ gemeinsam mit den Kohlenstoffatomen, an die sie gebunden sind, ein ankondensiertes carbocyclisches Ringsystem mit 5 bis 16 Kohlenstoffatomen;

n' gleich 0, 1 oder 2;

m' gleich 1.

5. Eine Verbindung nach Anspruch 4, dadurch gekennzeichnet, daß in der angegebenen Formel bedeuten:

$R_8$ und $R_{10}$ jeweils unabhängig voneinander ein Wasserstoffatom oder eine Methyl-, Ethyl-, Methoxy-, Hydroxy-, Ethoxy-, Phenyl-, Phenoxy-, p-Anisyl-, 2,5-Dimethoxyphenyl-, p,N,N-Dimethylaminophenyl- oder Diarylaminogruppe oder eine Gruppe der Formeln:

$$-\!(CH{=}CH)_{\overline{n'}}CH{=}\underline{A}_1 \text{ oder } -\!(CH{=}CH)_{\overline{n'}}\underline{A}_2;$$

wobei $\underline{A}_1$ und $\underline{A}_2$ die in Anspruch 2 angegebene Bedeutung haben; und

X für $—BF_4$, $—ClO_4$, $—CF_3$, $—SO_3$, $—FSO_3$, $—PF_6$, $—CH_3SO_3$, Chlor, Brom oder Jod steht.

6. Photoleitfähige Zusammensetzung mit einem Elektronen spendenden organischen Photoleiter und einer sensibilisierenden Menge einer Telluropyryliumverbindung nach Ansprüchen 1, 2, 3, 4 oder 5, in der die Verbindung in einer Menge von 0,001 bis 30% , bezogen auf das Gewicht der Zusammensetzung, vorliegt.

7. Photoleitfähige Zusammensetzung nach Anspruch 6, ion der der Elektronen spendende Photoleiter ein tertiäres Amin, gegebenenfalls ein Triarylamin ist.

**Revendications**

1. Composé de telluropyrylium ayant la structure:

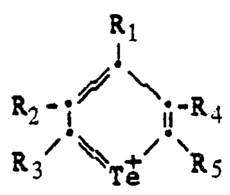

$(X^-)_m$; I.

dans laquelle

$R_1$, $R_3$ et $R_5$ représentent chacun indépendamment un atome d'hydrogène, ou un groupe alkyle, alkylamino, dialkylamino, alkoxy, alkylaryle, aryle ou aryloxy, dans lesquels chaque groupe alkyle ou alkoxy est un groupe hydrocarboné à chaîne droite ou ramifiée, substitué ou non, ayant de 1 à 16 atomes de carbone; et chaque groupe aryle ou aryloxy est un groupe phényle, naphtyle ou anthryle, substitué ou non; ou un groupe hétérocyclique ayant de 5 à 20 atomes de carbone, dans lequel les hétéroatomes

peuvent être un atome d'oxygène, de phosphore, d'azote, de soufre, de sélénium ou de tellure; ou un groupe

$$-(CH=CH)_n-CH=\underline{A}_1, \text{ ou } -(CH=CH)_n-\underline{A}_2$$

ou un groupe dicétonate zwitterionique de structure:

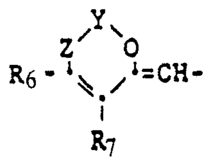 ;

$R_2$ et 4 représentent chacun indépendamment un atome d'hydrogène ou un groupe alkyle à chaîne droite ou ramifiée, substitué ou non, ayant de 1 à 16 atomes de carbone; ou un groupe phényle, naphtyle ou anthryle substitué ou non; ou bien $R_2$ et $R_3$, ou

$R_4$ et $R_5$, pris ensemble avec les atomes de carbone auxquels ils sont attachés, forment un système de cycles condensés carbocycliques mono ou polynucléaires de 5 à 20 atomes de carbone;

$R_6$ et $R_7$ représentent chacun indépendamment un atome d'hydrogène ou un groupe alkyle à chaîne droite ou ramifiée, substitué ou non, ayant de 1 à 16 atomes de carbone; ou un groupe phényle, naphtyle ou anthryle, substitué ou non; ou un groupe hetérocyclique substitué ou non, dans lequel l'hétéroatome est un atome d'oxygène, de phosphore, d'azote, de soufre, de sélénium ou de tellure; ou un groupe hétérocyclylidène méthyle mono ou polycyclique, dans lequel l'hétéroatome est un atome d'oxygène, de phosphore, d'azote, de soufre, de sélénium ou de tellure; ou $R_6$ et $R_7$ pris ensemble avec les atomes de carbone auxquels ils sont attachés forment un groupe condensé mono ou polycyclique, carbocyclique ou hétérocyclique, ayant de 5 à 20 atomes de carbone, dans lequel les hétéroatomes sont des atomes d'oxygène, de phosphore, d'azote, de soufre, de sélénium ou de tellure;

$\underline{A}_1$ représente un groupe hétérocyclique, mono ou polycyclique, conjugué, par l'intermédiaire d'une liaison méthinique ou polyméthinique, au cycle telluropyrylium, dans lequel les hétéroatomes sont des atomes d'oxygène, de phosphore, d'azote, de soufre, de sélénium ou de tellure, et le système cyclique a de 5 à 20 atomes de carbone;

$\underline{A}_2$ représente un atome d'hydrogène, un groupe amino, alkyle, alkylamino, dialkylamino, dialkylaminoaryle, ou alkoxy, dans lesquels chaque groupe alkyle ou alkoxy est un groupe hydrocarboné à chaîne droite ou ramifiée, substitué ou non, ayant de 1 à 16 atomes de carbone; ou un groupe aryle, arylamino ou diarylamino, substitué ou non, dans lesquels chaque groupe aryle est un groupe phényle, naphtyle ou anthryle; ou un groupe hétérocyclique, mono ou polycyclique, ayant de 5 à 20 atomes de carbone, dans lequel l'hétéroatome est un atome d'oxygène, de phosphore d'azote, de soufre, de sélénium ou de tellure, le dit groupe hétérocyclique étant conjugé, directement ou indirectement par l'intemédiaire d'une liaison polyméthinique, au noyau telluropyrylium;

n est un nombre de 0 à 5;

m est égal à 1, sauf quand $R_1$, $R_3$ ou $R_5$ est un groupe zwitterionique; m est alors égal à 0;

X est anion;

Y est $BF_2$ ou $PF_4$;

Z est un atome d'oxygène ou de soufre.

2. Composé conforme à la revendication 1, dans lequel $R_1$, $R_3$ et $R_5$ représentent chacun indépendamment un groupe méthyle, éthyle, phényle, P-N,N'-diméthylaminophényle, p-anisyle, phénoxy, éthoxy, méthoxy; ou un groupe:

$$-(CH=CH)_n-CH=\underline{A}_1 \text{ ou } -(CH=CH)_n-\underline{A}_2;$$

ou un groupe dicétonate zwitterionique ayant la structure:

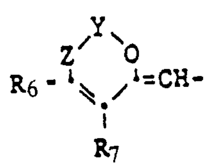 ;

$R_6$ et $R_7$ représentent chacun indépendamment un groupe méthyle, phényle, méthoxyphényle, p-N,N-diméthylaminophényle, aminophényle, pyridyle, oxazolyle, thiazolyle, sélénazolyle, pyranyle, thiapyranyle, sélénapyranyle, telluropyranyle ou oxaindolazinyle, ou $R_6$ et $R_7$ pris ensemble avec les atomes de carbone auxquels ils sont attachés, sont tels que définis à la revendication 1;

$\underline{A}_1$ représente un noyau oxazolylidène, thiazolylidène, sélénazolylidène, imidazolylidène, pyranylidène, thiapyranylidène, sélépyranylidène, telluropyranylidène, oxaindolazynilidène, benzoxazolylidene, benzo-

thiazolylidène, benzopyranylidène, benzothiapyranylidène, benzosélénapyranylidène, ou benzotelluropyranylidène;

$\underline{A}_2$ représente un atome d'hydrogène, ou groupe méthyle, méthoxy, éthoxy, phényle, diméthylaminophényle, ou diméthylamino, ou un noyau oxazolyle, 9-julolidyle, pyridyle, thiazolyle, sélénazolyle, imidiazolyle, pyryliumyle, thiapyryliumyle, sélénapyrylium, telluropyrylium, pyridinyle, furanyle, thiophényl, sélénophényle, tellurophényle, oxaindolazinyle, benzoxazolyle, benzothiazolyle, benzopyryliumyle, benzothiapyryliumyle, benzosélénapyryliumyle, benzotelluropyryliumyle ou naphtyle;

X représente $BF_4$, $ClO_4$, $SO_3$, $CF_3$, $CF_3SO_3$, $FSO_3$, $PF_6$, $CH_3SO_3$, chlore, brome ou iode.

3. Composé conforme á la revendication 1, dans lequel $R_2$ et $R_3$, ou $R_4$ et $R_5$, pris ensemble avec les atomes de carbone auxquels ils sont attachés, forment un noyau benzène.

4. Composé conforme à la revendication 3, dans lequel le noyau benzotelluropyrylium a la structure:

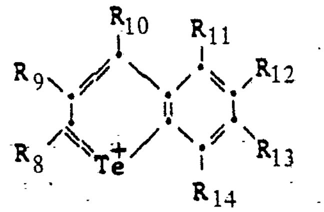

$(X^-)_{m'}$; III.

dans laquelle

$R_8$ et $R_{10}$ représentent chacun indépendamment un atome d'hydrogène ou un groupe alkyle, alkylamino, arylamino, alkoxy, aryloxy, dialkylamino, diarylamino,

$$-(CH=CH)_{n'}-CH=\underline{A}_1 \text{ ou } -(CH=CH)_{n'}-\underline{A}_2;$$

$R_9$ représent un atome d'hydrogène ou un groupe alklyle à chaîne droite ou ramifiée, substitué ou non, ayant de 1 à 16 atomes de carbone; ou un groupe phényle, naphtyle ou anthryle, substitué ou non;

$\underline{A}_1$ et $\underline{A}_2$ sont tels que définis à la revendication 1;

$R_{11}$, $R_{12}$, $R_{13}$ et $R_{14}$ représentent chacun indépendamment un atome d'hydrogène ou d'halogène, ou un groupe alkyle, alkoxy, aryle, alkylamino ou arylamino; ou

$R_{11}$, et $R_{12}$, $R_{12}$ et $R_{13}$ ou $R_{13}$ et $R_{14}$ pris ensemble avec les atomes de carbone auxquels ils sont attachés, forment un système carbocyclique condensé ayant de 5 à 16 atomes de carbone;

n' est égal à 0,1 ou 2;

m' est égal à 1.

5. Composé conforme à la revendication 4, dans lequel $R_8$ et $R_{10}$ représentent chacun indépendamment un atome d'hydrogène ou un groupe méthyle, éthyle, méthoxy, hydroxy, éthoxy, phényle, phénoxy, p-anisyle, 2,5-diméthoxyphényle, p-N,N-diméthylaminophényle, diarylamino,

$$-(CH=CH)_{n'}-CH=\underline{A}_1 \text{ ou } -(CH=CH)_{n'}-\underline{A}_2$$

$\underline{A}_1$ et $\underline{A}_2$ sont tels que définis à la revendication 2,

X représente $BF_4$, $ClO_4$, $CF_3$, $SO_3$, $FSO_3$, $PF_6$, $CH_3SO_3$, chlore, brome ou iode.

6. Composition photoconductrice comprenant un photoconducteur organique donneur d'électron et une quantité sensibilisatrice d'un composé telluropyrylium tel que défini aux revendications 1, 2, 3, 4 ou 5, dans laquelle ledit composé est présent à une teneur comprise entre 0,001 et 30% du poids de la composition.

7. Composition photoconductrice conforme à la revendication 6, dans laquelle le photoconducteur donneur d'électron comprend une amine tertiaire, éventuellement une triarylamine.